# EUROPEAN PATENT SPECIFICATION

(11) **EP 3 003 417 B1**
(45) Date of publication and mention of the grant of the patent: **21.08.2019**
(21) Application number: 14811959.7
(22) Date of filing: 02.06.2014
(51) Int. Cl.: A61L 27/38, A61L 27/50, A61L 27/52, A61L 27/58

(54) **COMPOSITIONS FOR BLOOD VESSEL TISSUE REPAIR**
ZUSAMMENSETZUNGEN ZUR BLUTGEFÄSSGEWEBEREPARATUR
COMPOSITIONS POUR LA RÉPARATION DES TISSUS DE VAISSEAUX SANGUINS

(30) Priority: 31.05.2013 US 201361829810 P
(43) Date of publication of application: 13.04.2016
(73) Proprietor: Xeltis BV, 5612 AR Eindhoven (NL)
(72) Inventor: SERINO, Francesco, Abu Dhabi (AE)
(74) Representative: FRKelly
(86) International application number: PCT/IB2014/002054
(87) International publication number: WO 2015/008152

(56) References cited:
- WO-A1-00/44808
- WO-A1-2012/028881
- WO-A2-2009/134991
- US-A- 4 902 289
- US-A1- 2010 125 330

## Description

### FIELD

The present disclosure relates to compositions and methods for blood vessel repair, tissue regeneration or engineering, in particular for treatment of diseased, occluded, or otherwise damaged blood vessels.

### BACKGROUND

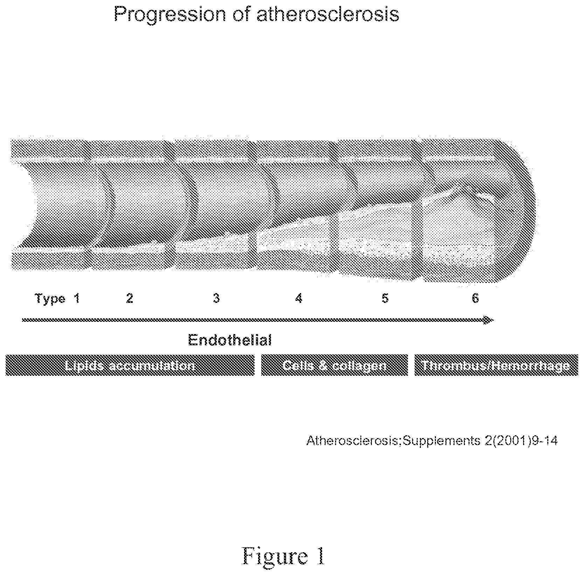

Atherosclerosis is the primary cause of cardiovascular disease (CVD). It is a progressive, chronic degeneration of the arteries which is initiated in the intima and eventually compromises the integrity of the entire vessel and its patency, obstructing blood flow and causing ischemia of the supplied organ. The intima is the inner most layer of an artery. It extends from the media to the lumen of the vessel and is composed of an internal elastic lamina and an endothelial cell layer. It is rich in connective tissue, especially proteoglycans. In atherosclerosis, the intima is thickened which causes narrowing of the lumen. Atherosclerotic plaques attaching to the already narrowed arterial lumen can, if not treated, lead to total occlusion of the vessel or may disrupt occluding the lumen distally (Figure 1).

Complicated atherosclerosis (lumen occlusion) produces a spectrum of manifestations, from unstable angina to myocardial infarction, from critical limb ischemia to gangrene of the lower extremities, from transient ischemic attack to ischemic stroke of the brain. Worldwide statistics have established that atherosclerosis is affecting younger people, including those of working and reproductive age. The organs affected by atherosclerosis are not just limited to the brain and heart, but all other organs of the body as well, including the kidneys, the gastrointestinal tract and the lower limbs. The population at the highest risk of this disease is diabetic patients, the largest population of patients to receive lower extremity amputation due to peripheral artery obstruction, a procedure which even today takes place every 30 seconds.

The objective of surgical therapy of chronic arterial obstruction is the relief of the consequent ischemia and the prevention of the degeneration organs or tissues which are fed by the arteries, by re-establishing arterial flow, i.e. to revascularize the ischemic heart, brain, arms, legs and other organs to avoid their loss.

The principal technique for revascularization established in the 1950's is to "bypass" the obstruction by inserting a synthetic tubular graft or to transplant a native vein to reroute the arterial flow past the obstruction, leaving the diseased arterial segment to degenerate in isolation. In the 1970's a new concept was introduced and rapidly developed into the "angioplasty" technique, which aims to re-channel the obstructed artery by inflating a balloon at high pressure to displace the intimal plaque. This action stretches the entire artery and compresses the plaque in order to establish a larger vessel lumen. Such plaque breaks down as a consequence of angioplasty, sometime causing debris flowing downward and occluding smaller vessels with definitive target tissue damage (e.g., the foot, brain, or heart). In some cases, debris detaches from the underlying media occluding the same vessel we are trying to reopen. The latter phenomena is generally adjusted using intraluminal devices called "stents", which forces the wall to remain opened by a radial force delivered by stiff struts. All revascularization techniques offer only a temporary solution to the disease, because with bypass surgery, native veins or synthetic grafts occlude within years and in angioplasty, the recurrence of the disease in the treated segment (restenosis) affects the long term patency of the vessel. Moreover, none of these techniques or their supporting theories aims to heal or regenerate the native artery.

WO2009134991 discloses a tubular construct for a tissue engineered blood vessel comprising a scaffold comprising poly(p-dioxanone) and one or more or of cells, cell sheets, cell lysate and minced tissue.

WO2012028881 discloses an injectable composition that can be solidified to form a scaffold and that comprises (i) polymeric particles that comprise a blend of poly (lactic-co-glycolic acid) and polyethylene glycol and (ii) hydrogel.

US2010125330 discloses a completely biodegradable graft, comprising: an outer absorbent layer comprising a blend of a biodegradable polymer and a lactam; and an inner layer comprising a biodegradable polymer of a molecular weight greater than the biodegradable polymer of the outer absorbent layer.

US4902289 discloses a multilayer blood vessel prosthesis comprising: a first inner conduit defining a relatively smooth walled lumen, said inner layer is crosslinked aminopolysacchride ride having a mean pore diameter below 10 microns, a wall thickness between 0.1 to 5.0 mm, and a number average molecular weight between crosslinks of between 2000 and 12,000 daltons and a second outer layer on said first layer, said outer layer formed a crosslinked freeze dried collagen-aminopolysascharide polymer having a mean pore diameter of at least 50 microns and a thickness of at least 1.0 mm.

WO2000044808 discloses a biomaterial formed by combining two or more precursor components of a biomaterial under conditions that allow polymerization of the two components, wherein said polymerization occurs through self selective reaction between a strong nucleophile and a conjugated unsaturated bond or a conjugated unsaturated group, by nucleophilic addition, wherein the functionality of each component is at least two, said biomaterial does not comprise unprocessed albumin, and said unsaturated bond or group is not a maleimide or a vinyl sulfone.

Atherosclerosis is not only the primary cause of CVD and the leading cause of death in developed countries, but is also a unique disease in that it is diffuse (affecting the entire CV system), multifocal (clinical eruption can affect multiple organs in a single patient) and recurrent.

What is needed, therefore, is a tissue engineering approach offering a biocompatible and definitive treatment for patients affected by any of the manifestations of CVD, by regenerating the diseased segments of affected blood vessels.

### SUMMARY

The present invention provides a synthetic layer scaffold for rebuilding a diseased intima layer of an artery as is claimed and suitable for tissue engineering and having an element for adhesion of a host cell. The synthetic layer provides a scaffold along an inner surface of a blood vessel for guiding tissue regeneration. In some embodiments the synthetic layer can fill atherosclerotic plaque or arterial wall defects in order to rebuild a smooth and homogeneous inner arterial layer. In a further embodiment this synthetic layer can homogenize with the atherosclerotic plaque in order to prevent or mend its rupture or breakdown. The composition can comprise a hydrogel which forms the synthetic layer in the blood vessel *in situ.* The composition can also comprise a synthetic layer that is preformed as a hollow cylindrical composite. In some embodiments, the synthetic layer comprises a plurality of hollow tubular composites. The synthetic layer is a polymer layer. The polymer is biocompatible. The polymer is resorbable. The synthetic layer acts as a synthetic intimal layer in the blood vessel. In another embodiment the synthetic layer acts as an internal elastic lamina. In another embodiment, the synthetic layer provides a substrate for repair of the blood vessel.

The synthetic layer of the present invention can have an inner surface and an outer surface. The outer surface of the synthetic layer adheres to the inner surface of the blood vessel. In some embodiments, the outer surface of the synthetic layer comprises RGD peptides. In another embodiment, the outer surface provides a substrate for formation of a blood vessel medial layer. In one embodiment, the inner surface provides a substrate for formation of a tissue layer comprising endothelial cells or progenitor cells. In a further embodiment, the above tissue layer is an intimal layer of the blood vessel. In another embodiment, the tissue layer is an internal elastic lamina of the blood vessel.

The synthetic layer of the present invention is porous. In another aspect, the synthetic layer is elastic. In still another aspect, the synthetic layer is resistant to shear stress.

In one embodiment, the element for adhesion of a host cell comprises a signaling element. In another embodiment, this signaling element is a bioactive polymer comprising at least one adhesion domain. The element for adhesion of a host cell comprises a plurality of nanoscale pores. In some embodiments, the element for adhesion of a blood vessel tissue cell comprises at least one pore.

In some embodiments, the synthetic layer is a hydrogel. In some embodiments, the synthetic layer is thermally or chemically sensitive. In some embodiments, the synthetic layer undergoes a phase change at body temperature. In some embodiments, the synthetic layer jellifies or solidifies at body temperature. In some embodiments, the synthetic layer is a liquid at a temperature below body temperature.

In one embodiment, the synthetic layer comprises a mesh comprising a polymer. In some embodiments, the synthetic layer comprises multiple meshes comprising a polymer. The polymer is biocompatible. The polymer is resorbable. The polymer is bioresorbable.

Also disclosed is a method of tissue engineering a blood vessel, comprising identifying a blood vessel in need of tissue repair or engineering; and inserting a composition of the invention (e.g., a structured scaffolding element) into the blood vessel. In one method, the method regenerates the intimal layer of the blood vessel. In some methods, the synthetic layer controls the proliferation of the cells of the native vessel media. In some methods, the method is performed *in vivo.* In other methods, the method is performed *ex vivo.*

Also disclosed herein is a method of treating a patient having cardiovascular disease, the method comprising identifying a blood vessel in the patient in need of tissue repair or engineering, coring the blood vessel, and inserting a composition of the invention (e.g., a structured scaffolding element) into the blood vessel. In one aspect, the coring is a method for at least partial removal of a plaque from the blood vessel. In some aspects, the coring is a radical debulking of the blood vessel, exposing the medial layer along the inner surface of the blood vessel. In another aspect, the exposed medial layer contacts the composition after insertion of the composition of the present invention. In another aspect, the coring could be a balloon angioplasty, endoatherectomy, or radical debulking. In some methods, the cardiovascular disease treated by a method is atherosclerosis. In some methods, the method is performed *in vivo.* In other methods, the method is performed *ex vivo.* The methods of tissue regeneration or engineering could be performed both *in vivo* or *ex vivo,* for example in a vessel, tissue or organ to be transplanted.

Also provided herein is a synthetic layer scaffoldsubstrate for rebuilding a diseased intima layer of an artery as is claimed and comprising a biocompatible, bioresorbable synthetic layer, wherein the synthetic layer comprises an element for adhesion to a cell or tissue, wherein the synthetic layer provides a scaffold for guiding tissue formation. In some embodiments, the synthetic layer is a hydrogel. The synthetic layer is porous. In one embodiment, the synthetic layer comprises binding moieties for binding to the cell or tissue. In one embodiment, the binding moieties comprise polypeptides capable of binding to the cell or tissue. In some embodiments, the composition is in liquid form upon delivery, and wherein the composition undergoes a phase change after delivery to a gel or solid. In some embodiments, the composition is temperature-sensitive. In some embodiments, the composition solidifies at body temperature. In some embodiments, the composition solidifies upon addition of a chemical.

Also disclosed herein is a method of repairing or engineering a tissue, the method comprising identifying a tissue in need of tissue repair or engineering and delivering the composition of the present invention to the surface of the tissue. In one method, the composition delivered is in liquid form before the delivery and in solid or gel form after the delivery.

### BRIEF DESCRIPTION OF THE FIGURES

FIG. 1: Stages of the progression of atherosclerosis in a blood vessel.

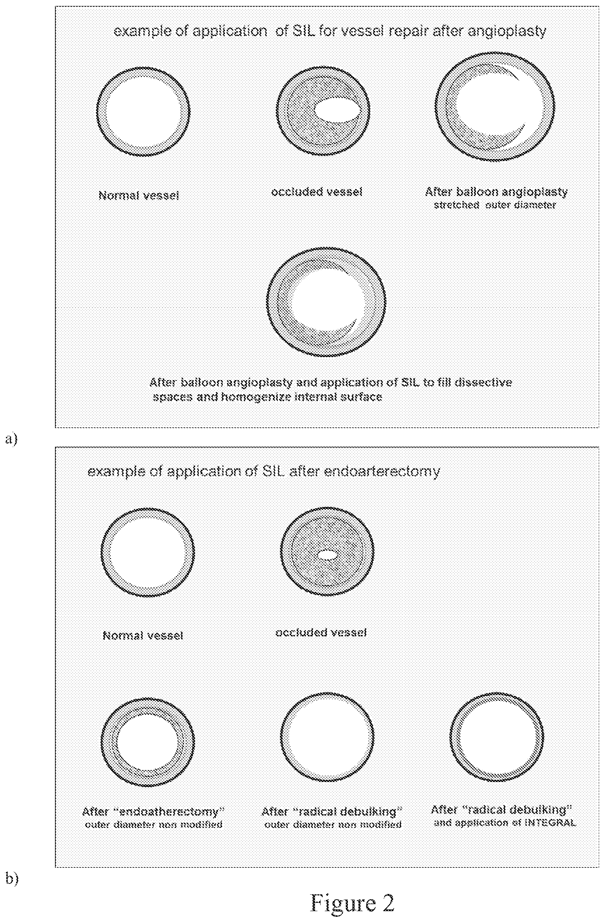

FIG. 2: Cross-sectional representation of blood vessels in normal, occluded, and treated phases. The diagram provides a comparison between the blood vessel after conventional balloon angioplasty (plaque rupture to enlarge vessel lumen) (a) and the blood vessel after radical endo-arterectomy or debulking (plaque removal) (b) and in both representation the effect of application of the synthetic intimal layer (SIL) or synthetic intimal elastic layer (SIEL) to achieve immediately a smooth inner surface and a reconstituted intima once the SIL would be replace by a regenerated intima.

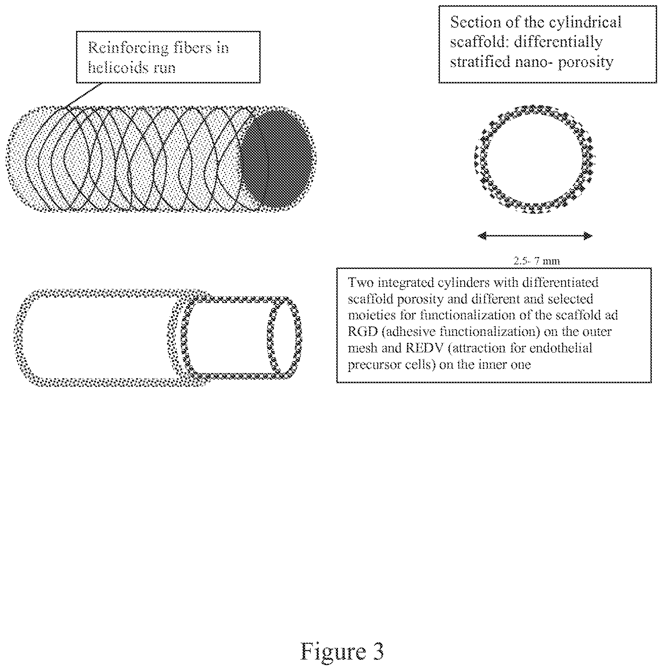

FIG. 3: Representation of the inner and outer layers of the cylindrical layer and reinforcing fibers in the cylindrical layer.

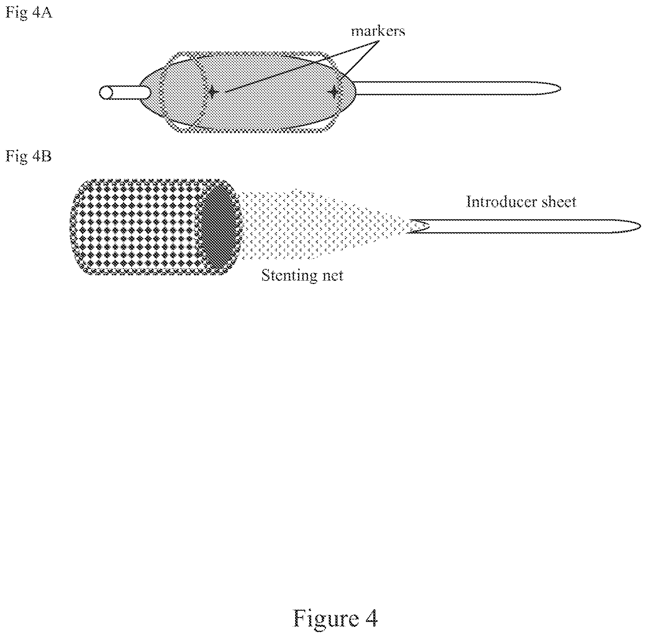

FIG. 4: Diagram of the insertion of the preformed cylindrical synthetic layer as a single or stratified meshes into a vessel. Figure 4A shows insertion of the cylindrical synthetic layer via a balloon catheter with radio opaque markers used to guide the layer. Figure 4B shows the insertion of the cylindrical layer into a vessel using a stenting net.

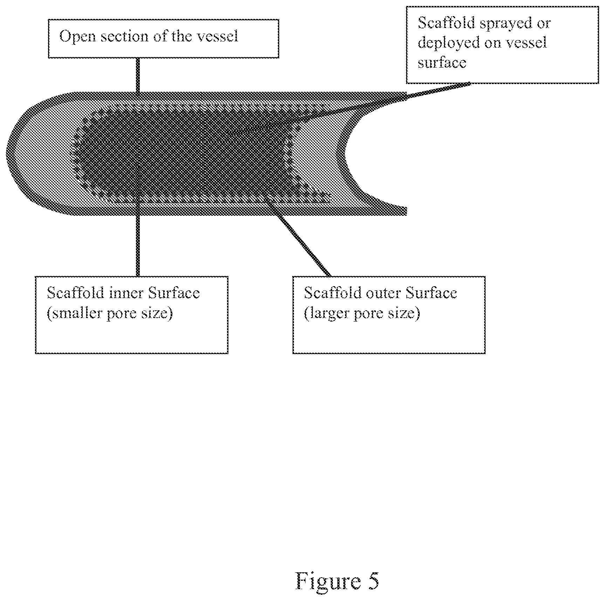

FIG. 5: Diagram of the deployment of a hydrogel formulation of the scaffold in liquid phase, by spraying onto a vessel surface. Inner and outer layers of the solidified scaffold are depicted. Relative pore size of the layers is represented, with the outer layer (attached to the inner wall of the vessel) comprising larger pores than the inner layer.

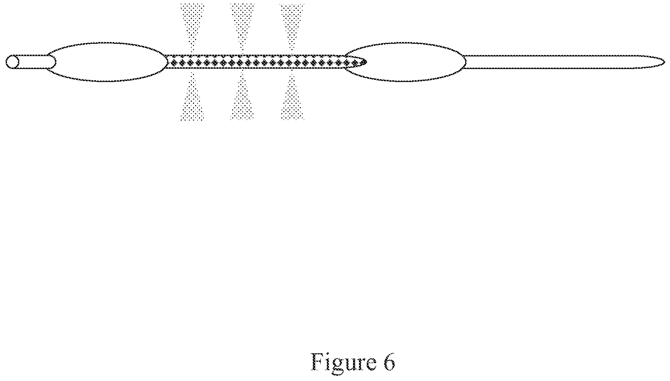

FIG. 6: Diagram of the delivery system of a temperature sensitive hydrogel formulation. The scaffold is delivered in liquid phase by a windowed catheter segment between two double compliant balloons. This designed delivery system allows safe delivery of a thermal or self setting hydrogel formulation of a scaffold, while blocking the blood flow in the arterial segment to be treated, to allow sufficient time for a phase change, solidification or reaction of the scaffold hydrogel after delivery to the blood vessel.

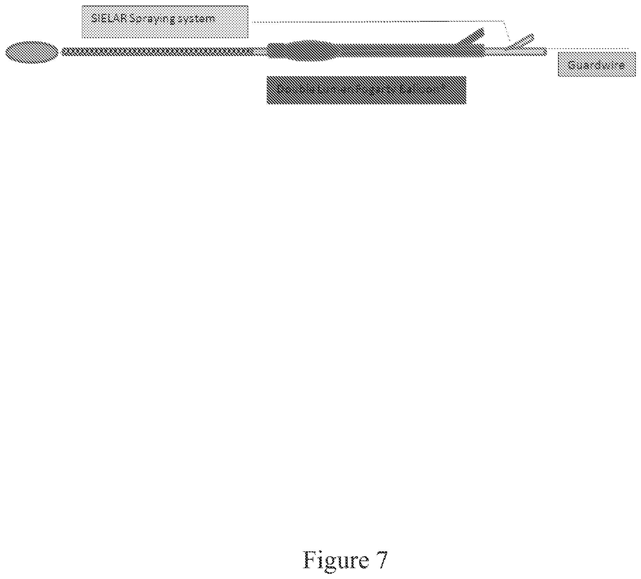

FIG. 7: Diagram of an alternative liquid phase scaffold delivery system using a Double Lumen Fogarty Balloon, a SIEL AR (Synthetic Intimal Elastic Layer for Arterial regeneration) Spraying system, and a Guardwire

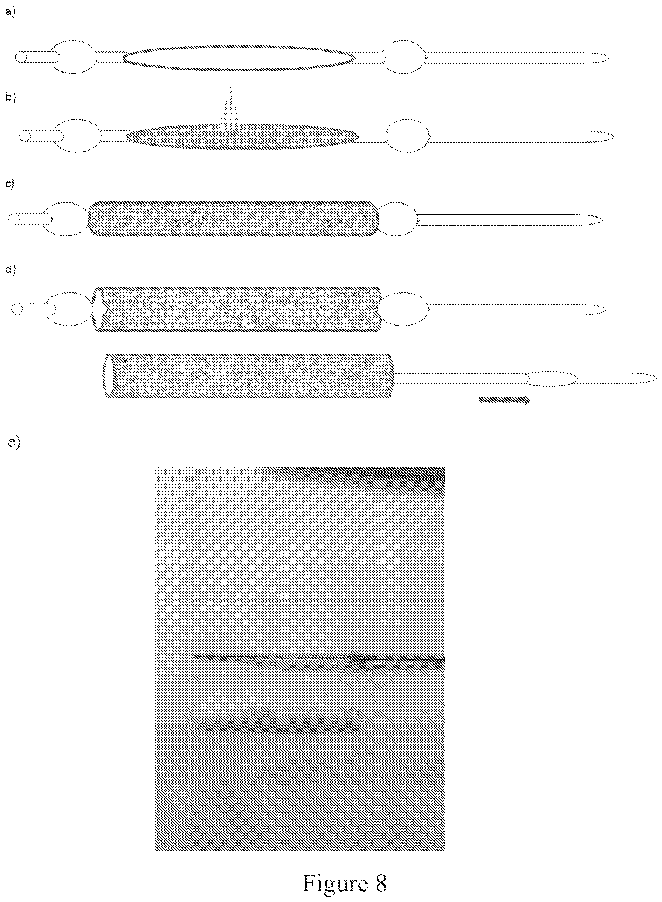

FIG. 8: Diagram of storage and delivery of the hydrogel scaffold, which may be, for example, temperature sensitive, self-setting, or chemically-setting once deployed in the blood vessel. In part A), deployment of an engineered scaffold on a delivery balloon catheter for release in a target vessel is shown. In part B) the scaffold is formed as a hydrogel and deployed by spraying or dipping or layering on the delivery balloon. After a selective process for storage (e.g., lyophilization, dehydration, or solidification), the scaffold is delivered by inflating a balloon catheter appropriately sized for the target vessel as shown in part C). The surface of the scaffold comprises specific elements for attachment to the blood vessel, allowing total detachment from the delivery balloon material transfer to the vessel inner wall. Total transfer of the scaffold to the vessel allows retraction of a downloaded delivery system, as shown in part D). Part E) shows a scaffold in hydrogel formulation, colored with methylene blue and deployed in a paper cylinder to proof homogeneous material release and transfer.

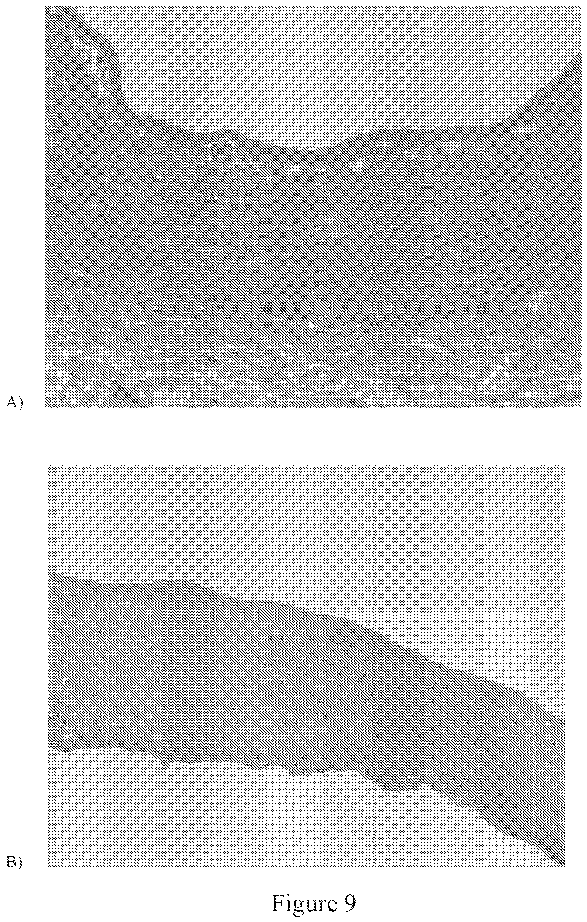

FIG. 9: Cross-sectional image of a rabbit aorta with the fiber mesh of a bioresorbable scaffold (in this case recombinant elastin like polymer delivered and self retaining on the native rabbit aorta. A) Polymer mesh applied without pressure on the native aorta, showing efficient superadhesive properties of the RGD moieties on the outer suface of the scaffold to the inner surface of the aorta. B) Ploymer mesh deployed with a balloon inflated at low presure (2 atm) showing complete and total adhesion to the arterial wall. This picture showes the super adhesivity of the scaffold and its ability to produce an inner arterial smooth layer.

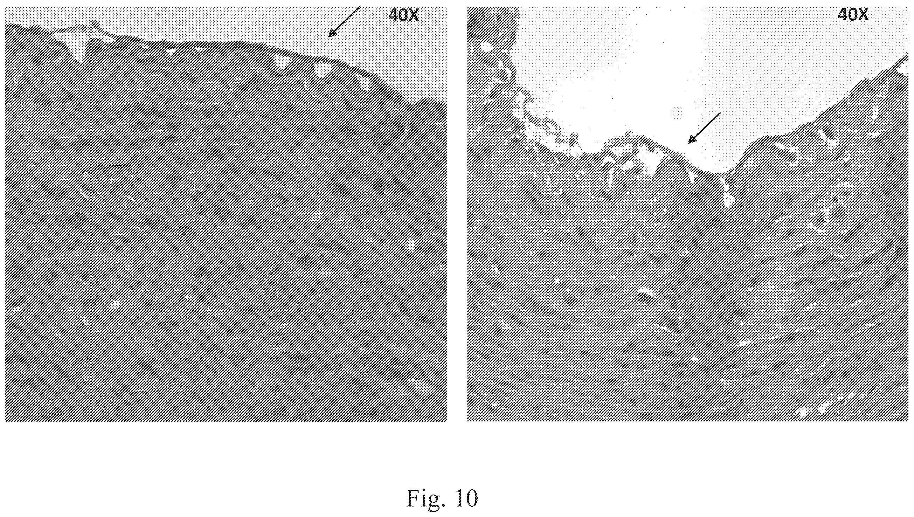

FIG. 10: 40X magnified image of RGD-enhanced hydrogel scaffold (with an elastin-like polymer), deployed on native rabbit aorta. Deployment was performed by a balloon catheter, at low inflation pressure in absence of stretching force applied on tissue.

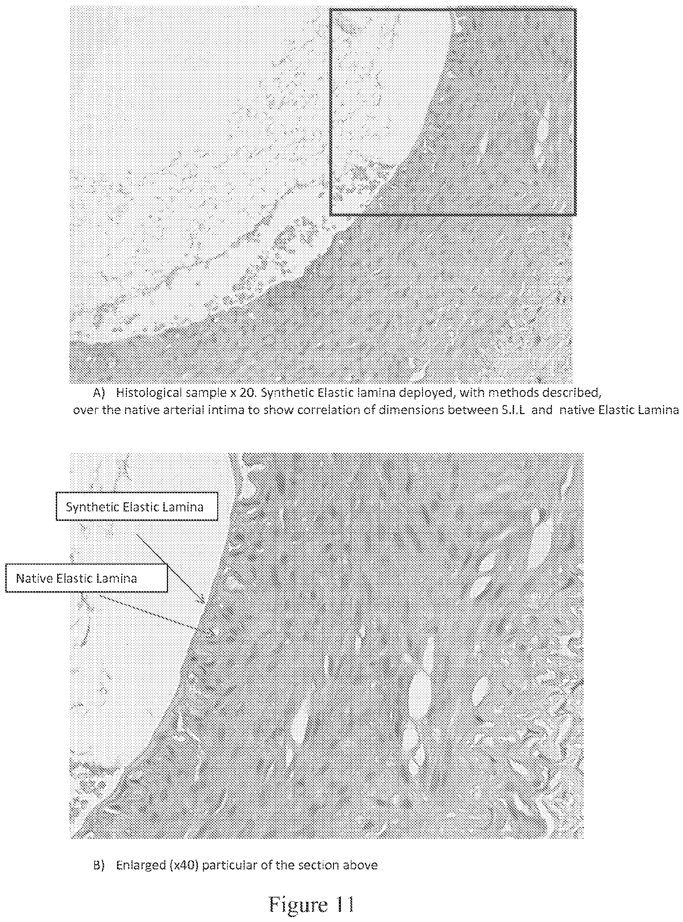

FIG 11: 20X (a) and 40X particular (b) of a rabbit aortic histology section after application of a thin version of the synthetic intima elastic layer. In this example it is shown the capability to produce, deliver and retain of a Synthetic intima/elastic layer of the same thickness as the native one.

### DETAILED DESCRIPTION

Unless otherwise defined herein, scientific and technical terms used in connection with the present invention shall have the meanings that are commonly understood by those of ordinary skill in the art. Further, unless otherwise required by context, singular terms shall include the plural and plural terms shall include the singular. The methods and techniques disclosed are generally performed according to conventional methods well known in the art and as described in various general and more specific references that are cited and discussed throughout the present specification unless otherwise indicated.

The following terms, unless otherwise indicated, shall be understood to have the following meanings:
"Biocompatible" as used herein refers to the ability of a material to perform with an appropriate host response in a specific application. An appropriate response includes one in which adverse or unwanted affects are absent or substantially mitigated in the host.
"Resorbable" as used herein refers to a substance which can undergo resorption, as this term is defined hereinbelow. When the resorption process takes place in a biologic system, the substance is referred to as "bioresorbable."
The term "resorption," as used herein describes a loss of a substance through chemical, biological and/or physiologic processes. Typically this term is used herein and in the art to describe such a process which involves decomposition of a substance by, e.g., chemical or physical break-down, such as dissolution, hydrolysis and/or phagocytosis, which may be followed by absorption and/or excretion of the breakdown products by the body via, for example, metabolism. The term resorption is therefore often referred to herein and in the art as "bioresorption."
The term "synthetic" or "synthetic component" as used herein refers to a component that does not normally exist in nature. Generally, synthetic components are not normally present in a native blood vessel. A synthetic component may be part of a tissue engineering scaffold and/or a tissue engineered blood vessel, as described herein, that may optionally include a natural component (as defined below). Synthetic components may be elastomeric or tensile in nature. In one example, the synthetic components do not mimic the biomechanical behavior of native blood vessels.
The term "natural component" as used herein refers to a substance that exists in nature or is derived from a substance that exists in nature, regardless of its mode of preparation. Thus, for example, a "natural component" may be a native polypeptide isolated and purified from its native source, or produced by recombinant and/or synthetic means. Natural components may be present in a native blood vessel and therefore have the potential to exhibit native vessel-like properties with respect to mechanical and cellular behavior. In certain embodiments, natural components may be elastomeric or tensile in nature.
"Blood vessel tissue cell" as used herein refers to any cell which makes up the tissue of a blood vessel. For example, this may refer to endothelial cells and progenitor cells in the intimal layer of the blood vessel. In another example, blood vessel tissue cells refer to cells of the medial layer of the blood vessel.
The term "scaffold" as used herein refers to a three-dimensional supporting structure for attaching to and/or growing cells or tissues.
The term "hydrogel" as used herein refers to a semisolid composition constituting a substantial amount of water, and in which polymers or mixtures thereof are dissolved or dispersed.
The term "cylindrical" as used herein is of or pertaining to an approximate shape of a cylinder.
The term "functionalization" refers generally to the process of modification of scaffold prototypes, transforming them into "smart", biologically active platforms, capable of selecting, attracting and homing specific cells onto specific segments.
The "intimal layer" (or tunica intima) of the blood vessel is the innermost layer in an unmodified, healthy blood vessel. It is made up of one layer of endothelial cells and is supported by an internal elastic lamina. The endothelial cells are in direct contact with the blood flow in an unmodified, healthy blood vessel.
The "medial layer" (or tunica media) is the middle layer of an artery or vein. It is made up of smooth muscle cells and elastic tissue, and it lies between the tunica intima on the inside and the tunica externa on the outside.
The term "signaling element" as used herein, refers to an element that is capable of binding to or recruiting a selected cell or cell type. For example, a signaling element as part of a synthetic layer can bind to or recruit cells of a pre-selected type to predictably form a tissue layer.
The term "bioactive" as used herein, refers to a molecule that elicits or affects a biological event.
The term "cardiovascular disease" (CVD) generally refers to a number of diseases that affect the heart and circulatory system, including aneurysms; angina; arrhythmia; atherosclerosis; cardiomyopathies; cerebrovascular accident (stroke); cerebrovascular disease; congenital heart disease; congestive heart failure; coronary heart disease (CHD), also referred to as coronary artery disease (CAD), ischemic heart disease or atherosclerotic heart disease; dilated cardiomyopathy; diastolic dysfunction; endocarditis; heart failure; hypertension (high blood pressure); hypertrophic cardiomyopathy; mitral valve prolapse; myocardial infarction (heart attack); myocarditis; peripheral vascular disease; rheumatic heart disease; valve disease; and venous thromboembolism. As used herein, the term "cardiovascular disease" also encompasses reference to ischemia; arterial damage (damage to the endothelial lineage) due to physical damage (endoarterectomy, balloon angioplasty) or as a result of chronic damage (including atherosclerosis); myocardial damage (myocardial necrosis); and myonecrosis. In general, any physiological or pathophysiological condition that elicits a neoangiogenic response is encompassed by the term "cardiovascular disease" as used herein.
As used herein, the term "atherosclerosis" refers to forms of vascular disease characterized by the deposition of atheromatous plaques containing cholesterol and lipids on the innermost layer of the walls of large and medium-sized arteries. Atherosclerosis encompasses vascular diseases and conditions that are recognized and understood by physicians practicing in the relevant fields of medicine. Atherosclerotic cardiovascular disease, including restenosis following revascularization procedures, coronary artery disease (also known as coronary heart disease or ischemic heart disease), cerebrovascular disease including multi-infarct dementia, and peripheral vessel disease including erectile dysfunction, are all clinical manifestations of atherosclerosis and are therefore encompassed by the terms "atherosclerosis" and "atherosclerotic disease."
"Coring" refers to any method used to hollow or increase the size of the inner part of an object, such as its core. In the context of coring a blood vessel, the term is meant to encompass any method used to hollow a blood vessel, or to increase the area of the cross-sectional void of a blood vessel. It can be also referred as "atherectomy" or "endoatherectomy", meaning the coring or circumferential, progressive, ablation of the plaque. This could result in a decreased blood pressure per rate of blood flow through the blood vessel. These methods could also be used to prepare a blood vessel for implantation of a composition or device for regeneration of a blood vessel. Examples of methods to core a blood vessel include balloon angioplasty, surgical or close or endovascular endoatherectomy, and radical atherosclerotic plaque debulking. In one method, coring refers to the use of an appropriately modified atherectomy device to remove a diseased segment and also to its use to channel and shape the scaffold cast.
The terms "subject" or "patient" are used interchangeably herein and include, but are not limited to, an organism; a mammal, including, e.g., a human, non-human primate, mouse, pig, cow, goat, cat, rabbit, rat, guinea pig, hamster, horse, monkey, sheep, or other non-human mammal; and a non-mammal, including, e.g., a non-mammalian vertebrate, such as a bird (e.g., a chicken or duck), an amphibian and a fish, and a non-mammalian invertebrate.

The disclosure discloses a device for tissue engineering blood vessels for growth, repair, or regeneration of blood vessels. The device provides a bioactive, selectively porous, bioresorbable, preshaped or *in situ* structured scaffold, which guides the repair of the inner layer of the intimal layer of the blood vessel. The device guides the engineering of a new intimal layer of the blood vessel. The scaffold is designed to be repopulated by the cells from the healthy medial layer along the outer surface and relined by endothelial or progenitor cells along the inner surface. The scaffold induces controlled migration of specific endothelial cell and smooth muscle cells by selective signaling and increases material/host adhesion via a 3-dimentional bio-inspired morphology. The scaffold is resorbed progressively when the tissue in-growth is complete.

In certain embodiments, the scaffold material comprises natural or synthetic, composite and/or functionalized materials selected according to its biocompatibility and regenerating capacity for tissue or blood vessel growth, repair, or regeneration. The material may comprise a hydrogel for in situ gelling and secondary modeling. The material may comprise a composite for application as a pre-shaped device. The material may be a combination of a hydrogel and a pre-formed composite device.

Methods for tissue engineering blood vessels for growth, repair or regeneration of blood vessels are also disclosed. In one method, the method provides an approach for the treatment of cardiovascular atherosclerotic occlusive disease by ablation and regeneration of the diseased inner arterial intima.

In some embodiments, the surfaces of the layers of the scaffold are functionalized. Functionalization can be used to increase the adhesiveness of the surface to extracellular matrix and cellular adhesion molecules (i.e., endothelial, endothelial precursors, and smooth muscle cells). Cellular adhesion molecules can come from the pool of circulating blood cells. For example, smooth muscle cells are expected to migrate from the remaining healthy part of a medial layer. One such functionalization is the use of bioactive polymers engineered to express RGD (Arg-Gly-Asp) or REDV (Arg-Glu-Asp-Val) based adhesion domains and the application of appropriate nanometer-size porosity to bioresorbable substrates. Toward this aim, devices are designed for enhancing endothelial cell adhesion. In one embodiment, both the inner and the outer surfaces of the scaffolds are functionalized.

In an aspect of functionalization, a scaffold for material/host tissue adhesion is provided. This functionalization prevents misplacement of a blood vessel tissue engineering scaffold. A micro-nanometer-sized porosity is provided to the bioresorbable substrates. This aspect is used for improving cell adhesion, such as endothelial cell adhesion. Functionalization of the scaffold can promote intimal tissue regeneration. In one case, the scaffold is capable of being safely and efficiently implanted in arteries, maintaining appropriate mechanical and elastic properties.

The blood vessel tissue scaffolds described herein are biocompatible scaffolds, which are porous (macroporous or microporous or nanoporous) and that can provide controlled morphological and material-based gradients. The layers used in the scaffolds can have a structure that provides organization at the microstructure level that facilitates cellular invasion, proliferation, and differentiation that can ultimately result in regeneration of wholly or partially functional tissue. The layer(s) of the tissue scaffold have a microstructure that can for example permit tissue ingrowth, tissue repair, tissue regeneration, and cell based research for therapeutic agent discovery. In particular, the scaffold provides surface(s) that have been formed with domains that interface with living cells to control growth.

The blood vessel tissue scaffolds described herein include cell openings (e.g., cell openings defining pores in one or more surfaces) that vary in size and shape. Whether of a regular or irregular shape, the diameter of the cell opening can be between about 1 to about 10,000 microns. For example, cell openings can be from about 5 microns to 9,500 microns; from about 10 to 10,000 microns; from about 25 to about 7,500 microns; from about 50 to 5,000 microns; from about 100 to about 2,500 microns; from about 100 to about 5,000 microns; from about 250 to about 2,500 microns; from about 250 to about 1,000 microns; from about 500 to about 1,000 microns; from about 750 to about 1,000 microns; or ranges therebetween. The cellular openings can provide pathways for cellular ingrowth and nutrient diffusion. Porosities can be controlled and can range from about 10% to 95% porous. Because the cell openings and/or channels can have diameters in the range of microns, useful layers and scaffolds can be described as microporous.

The features of the blood vessel tissue scaffolds can be controlled to suit desired applications by selecting features to obtain the following properties: gradient along three axes for preferential cell culturing; channels that run through the scaffold for enhanced cell invasion, vascularization, and nutrient diffusion; micro-patterning of layers on the surface for improved cellular organization; tailorability of pore size and shape; anisotropic mechanical properties; composite layered structure with a polymer composition gradient to modify the cellular response to different materials; blends of different polymer compositions to create structures that have portions that degrade or resorb at different rates; layers blended or coated with bioactive agents (or "compounds") included but not limited to biological factors, growth factors, and the like; ability to make three dimensional structures with controlled microstructures; and assembly with other medical devices or agents to provide a composite structure.

In some embodiments, a biocompatible scaffold includes a substantially controllable pore structure. Characteristics selected from the group comprising composition, stiffness, pore architecture, and bioabsorption rate can be controlled. The scaffold can be made from an absorbable polymer. A blend of polymers can be applied to form a compositional gradient from one layer to the next. In applications where one composition is sufficient, the scaffold provides a biocompatible scaffold that may have structural variations across one or more layers that may mimic the anatomical features of the tissue. The structural variations can result in a variation in degradation across the scaffold. In particular, the scaffold does not rely on radial force to maintain its structure.

In one disclosure, a method for the repair or regeneration of blood vessel tissue includes contacting a first tissue with a scaffold pore gradient at a location on the scaffold that has appropriate characteristics to permit growth of the tissue. The concept of controlled transition in physical and chemical properties, and/or microstructural features in the scaffold can facilitate the growth or regeneration of tissue.

The scaffolds are particularly useful for the generation of tissue junctions between two or more layers of tissue. For a multi-cellular system, one type of cell can be present in one area of the scaffold and a second type of cell can be present in a separate area of the scaffold. Delivery channels can be utilized to position agents, compounds or cells in certain regions of the scaffold.

The materials used to produce the tissue scaffolds may be suitable for promoting the growth of either adhering, non-adhering cell lines, or any combination thereof.

In one case, the material used to produce the scaffold comprises a sheet. The sheet may be substantially planar. The material may be at least partially of a layered construction. In one case, the material comprises an inner and an outer layer, one layer having a higher absorption rate than the other layer. The inner and outer layer may be located adjacent to each other.

The outer layer interacts with or attaches to the inner surface of the diseased blood vessel media layer. The inner layer may promote regeneration of the intimal layer of the blood vessel, and the outer layer may promote ingrowths of the medial layer of the blood vessel into the scaffold and control medial layer cells proliferation. In one embodiment, the synthetic layer adheres to the cored medial layer.

Recruitment of the desired cells for tissue regeneration of the blood vessel may be achieved with signaling elements attached to the scaffold. This design encompasses a functionalized scaffold which increases the adhesion of the scaffold to the extracellular matrix and cellular (endothelial, endothelial precursors, and smooth muscle cells) adhesion molecules. Functionalization can be achieved, for example, with the use of bioactive polymers. Examples of bioactive polymers include RGD (Arg- Gly-Asp) and REDV (Arg-Glu-Asp-Val) based adhesion domains. The signaling elements are complemented by inclusion of appropriately sized pores in the scaffold. In one aspect, the pores are nanometer-sized.

In one embodiment the material is at least partially porous to promote tissue in-growth. The inner and outer layer may have different pore densities. The inner and outer layer may have different pore sizes. In one case, at least some of the pores form at least a partial gradient with varying density.

In another embodiment, the material is at least partially porous to promote tissue in-growth. The layers may have a higher pore density in select regions. The central region may have a higher pore density than the outer region. In one case, at least some of the pores form at least a partial gradient from one region to the next.

The material may comprise an anti-adhesion filler filling at least some of the pores. The material may comprise an anti-adhesion coating along at least part of the surface of the material. Alternatively, a material used to promote tissue attachment and bonding may be used with the scaffold.

As noted, the pores can have different dimensions, the layers can have different thicknesses, and the layers can have different compositions all of which vary the healing and biodegradation characteristics. In that instance, the method of making the scaffold can be carried out by: extruding a first biocompatible polymer to form a first layer or surface; forming pores in the first layer or surface; extruding a second biocompatible polymer to form a second layer or surface; forming pores in the second layer or surface; and attaching the first layer or surface to the second layer or surface to produce a tissue scaffold. The layers are each cylindrical in shape for regenerating or repairing blood vessel tissue. The tissue scaffold can be designed with controlled blood vessel tissue ingrowth and remodeling to permanently alter the mechanical properties of the tissue.

Where a layer is obtained, rather than made, the methods of making the tissue scaffold can simply require providing a given layer that is then attached (e.g., reversibly or irreversibly bound by mechanical or chemical forces), if desired, to another layer and/or processed to include one or more pores of a given size and arrangement. The single provided layer (or adherent multiple layers) can then be subjected to a process (e.g., laser ablation, die punching, or the like) that forms pores within the layer(s). Accordingly, any of the methods can be carried out by providing a given biocompatible layer, rather than by producing it by an extrusion or extrusion-like process. The layers used in the scaffold layers can also be produced using casting, injection molding, electrospinning, or dip coating techniques.

The blood vessel tissue scaffolds include a layer that is biocompatible. A biocompatible layer is one that can, for example, reside next to biological tissue without harming the tissue to any appreciable extent. As noted above, the layer(s) used in the scaffolds include pores (e.g., open passages from one surface of the layer to another) that permit tissue ingrowth and/or cellular infiltration.

The scaffolds can offer a combination of controlled porosity, high strength, and specific material content, and they may have one or more of the following advantages. They include pores or porous structures that stimulate tissue integration and reduce inflammation; they can reduce the risk of rejection with adjacent tissue (this is especially true with scaffolds having a smooth surface and atraumatic (e.g., smooth, tapered, or rounded) edges; they can simulate the physical properties of the blood vessel tissue being repaired or replaced, which is expected to promote more complete healing and minimize patient discomfort; their surface areas can be reduced relative to prior art devices (having a reduced amount of material may decrease the likelihood of an immune or inflammatory response). Moreover, scaffolds with a reduced profile can be produced and implanted in a minimally invasive fashion; as they are pliable, they can be placed or implanted through smaller surgical incisions. Methods may also produce scaffolds with improved optical properties (e.g., scaffolds through which the surgeon can visualize more of the underlying tissue). Practically, the micromachining techniques that can be used to produce the scaffolds are efficient and reproducible. The scaffolds described herein should provide enhanced biocompatibility in a low profile configuration while maintaining the requisite strength for the intended purpose.

The layer is made of, or includes, a biocompatible material that is biodegradable (i.e., it degrades within a human patient within a discernible period of time (e.g., within months or years)). The biocompatible material is at least partially absorbable by the body. The biocompatible material may comprise an absorbable polymer or copolymer such as Elastin like Polymers (ELP), polyglycolic acid (PGA), polylactic acid (PLA), polycaprolactone, polyhydroxyalkanoate, or polyfumarate and derivatives of the above polymers.

blood vessel tissue scaffolds can also include a biological material such as collagen, fibrin, or elastin. Biological materials such as these can be incorporated into one or more of the layers assembled into the scaffold (e.g., as a component of the layer or a coating thereon) or can be contained within one or more of the pores, pathways, or channels within the scaffold.

Biocompatible materials useful in the layers can include; absorbable polymers such as polyglycolic acid (PGA), polylactic acid (PLA), polycaprolactone, and polyhydroxyalkanoate, or copolymers thereof (e.g., a copolymer of PGA and PLA); or tissue based materials (e.g., collagen or other biological material or tissue obtained from the patient who is to receive the scaffold or obtained from another person.) The polymers can be of the D-isoform, the L-isoform, or a mixture of both.

In the case of a blood vessel tissue scaffold made from multiple layers, the various layers may be of the same or different materials. For example, in the case of an absorbable material, the material of the layers may be selected to have varying rates of absorption.

In one embodiment the biocompatible material has a plurality of cells. The biocompatible material may have a plurality of cells and one or more of the cells in the plurality of cells have a diameter, measured along the longest axis of the cell, of about 10 to about 10,000 microns. The biocompatible material may have a plurality of cells and one or more of the cells of the plurality are essentially square, rectangular, round, oval, sinusoidal, or diamond-shaped.

The thickness of one or more of the layers within the scaffold is about or less than about 0.004 inches. For example, the scaffold can be formed from one or more layers, which can be of the same or different thicknesses. For example, the layers can be 0.003 inches (75 µm) or less; 0.002 inches (50 µm) or less; or 0.001 inch (25 µm). As noted, a given scaffold can include more than one layer and the overall thickness of the scaffold can vary tremendously, depending on its intended application.

The tissue scaffold may comprise attachment regions, which may be adapted to receive sutures, staples or the like. In addition, the individual layers for the tissue scaffold may have alignment regions to ensure the pores in the layers match up properly.

Medical applications for the blood vessel tissue scaffolds described above may include but are not limited to repair and regeneration of diseased or damaged blood vessels. Referring to **Figure 1****,** a diseased blood vessel could refer to a blood vessel during any of the stages of atherosclerosis. Repair of the blood vessel could be initiated by coring of the blood vessel by several techniques, for example, balloon angioplasty, surgical or close or endovascular endoatherectomy, or radical atherosclerotic plaque debulking. A depiction of the results of some of these techniques is provided in **Figure 2**. Following angioplasty (plaque disruption by a balloon) or coring (plaque circumferential ablation of the plaque) of the atherosclerotic blood vessel, a blood vessel tissue scaffold as described is placed inside the blood vessel to fill dissection plans, even the surface or a disrupted plaque and promote regeneration and healing of the blood vessel tissues.

In some cases, tissue engineering of blood vessels can be used to supplement or to replace blood vessel grafts. In one approach, the disclosure, combines tissue engineering and designed biomaterials with minimally invasive approaches for blood vessel repair or therapy. The method also discloses an in situ solution to regeneration of a patient's arteries. In one aspect, the function of the regenerated vessel is preserved.

In one method, the method uses advanced biomaterials in an amorphous state to be shaped in situ by endovascular techniques. This could further reduce the complexity and the cost associated with current tissue engineering approaches, to solve the technical difficulties of shaping and delivering a preformed product or device into the vascular bed. Another advantage of this method is in the use of in vivo tissue engineering technology without the need for in vitro cell and tissue manipulation. In one aspect, the blood vessel tissue engineering device has the burst strength and fatigue strength to keep the arterial lumen patent since it is sustained by the remains of the arterial wall. In another aspect, the new endothelium is formed by the host's own cells and are not thrombogenic or immunogenic.

In one embodiment, the device provides a means of regenerating a functional, elastic intimal layer of diseased arteries after disruption, ablation or removal of their atherosclerotic plaque. Plaque partial or total removal will be accomplished by surgical or endovascular technology and the gap refilled by a resorbable scaffold which, once implanted will be seeded by circulating cells and filled by native arterial wall cells, eventually allowing a new arterial intimal layer to be formed in situ.

In some embodiments, the synthetic layer will be formed by a thin film of a scaffold made from a biocompatible, resorbable (natural or synthetic) polymer (BRP), suitable for human medical application, alone or in composite with other biodegradable natural or synthetic polymers to augment adhesiveness and support. BRP can be functionalized with addition of signaling peptides, proteins or drugs to achieve a "smart" feature, for which they may modulate cell homing, inflammatory reaction and long term biocompatibility. BRP will be composed, before (pre-formed in a laboratory) or within the deployment ("in vivo" application) a porous structure. An example of suitable material for the synthetic layer is Elastin Like Recombinant Polymers (ELRP).

In some embodiments, the hydrogel scaffold is in liquid form before delivery. In certain aspects of the invention, the hydrogel scaffold undergoes a phase change (e.g., solidifies or forms a gel layer) at a certain temperature (e.g., is "temperature sensitive" or "thermo sensible"). In a preferred embodiment, this temperature is body temperature. In some embodiments, the synthetic layer undergoes a phase change in the presence of a certain chemical or compound. In some embodiments, the synthetic layer undergoes a phase change after an amount of time after application to a tissue or vessel. In preferred embodiments, the hydrogel scaffold undergoes a phase change after application to the vessel or tissue. Once delivered into the vessel, the liquid may undergo a phase shift or solidification to form a more stable scaffold in the vessel as a hydrogel or solid. This liquid may automatically set once injected into the vessel, or the liquid may need to interact with an additional chemical to set after injection.

The synthetic layer may be stored in several forms, including in liquid form. The synthetic layer may also by lyophilized, dehydrated, or solidified for storage and/or preservation.

The following examples are for illustrative purposes and are not intended to limit the scope of the present invention which is defined by the appended claims.

### EXAMPLE 1

### Substitution of a diseased inner arterial layer with a Synthetic Intimal Layer

Current technology for mechanical closed endo-atherectomy allows the partial (central) removal of an obstructive plaque. This Example provides the possibility of expanding the atherectomy to a "radical" ablation of the plaque and the diseased intima by extensively ablating the atherosclerotic plaque up to the outer layer of the media. The diseased segment is then be replaced with a Synthetic Intimal Layer (SIL) or Synthetic Internal Elastica layer (SIEL) (**Figure 2**). SIL or SIEL is not intended to "stent" the artery. Conversely, it aims to replace the diseased and stiffened area with a soft and compliant intelligent scaffold. This is an example of *in situ* tissue engineering, where a smart degradable biomaterial drives the regeneration of tissue possessing native anatomy, whilst disappearing during the process of regeneration.

### EXAMPLE 2

### Production of a Synthetic Intimal Layer (SIL) or Synthetic Internal Elastica layer (SIEL) as a thin walled cylinder of biocompatible and bioresorbable scaffold material

The method provided below produces a thin (< 100 µm) bioactive, selectively porous, bioresorbable scaffold: the scaffold can be used either as an injectable hydrogel or as a composite in pre-shaped structures that can be deployed in contact with the healthy medial layer of the artery as a new internal elastic lamina.

Two different technical approaches can be used:
Approach A: A structured SIL or SIEL (in cylinders and sheets) made of composite materials can be deployed as a complete, still moldable, device in the endo-arterectomised artery.
Approach B: An injectable scaffold can be shaped in situ into SIL or SIEL through deposition or spraying and secondary modeling of the scaffold.

Features of this scaffold material include elasticity for compliance with arterial pulsatility, resistance to shear stress and progressive laminar absorption. In the pre-shaped embodiment, this scaffold is cylindrical or tubular and self retaining due to adhesive properties that will allow the maintenance of the structure with minimal thickness. In the second injectable embodiment, the same mechanical features of the scaffold is achieved by spraying and modeling of the "in vivo" gelling scaffold. The interconnection of the material, engineered with the desired porosity, with the host tissue secures the biomaterial to the host's arterial wall.

The deployment of the scaffold in pre-shaped or in an injectable form is performed by surgical and endovascular techniques.

In Approach A, tubular devices with engineered porosity and physiological mechanical properties and made of natural or recombinant polymers or proteins are developed. The tubular device is coupled with other synthetic or natural elements in composite materials. Different process technologies can be considered for the realization of cylindrically shaped scaffolds: the aim of the techniques is to create materials capable of withstanding long-term mechanical stress and capable of presenting bioactive elements. In some process technologies, the bioactive elements are present in recombinant proteins or material composites.

In Approach B, thermogeling, injectable, materials or polymer which may have bioactive elements are applied using two methods. In the first, spraying is performed using specially designed catheters and in situ gelling onto the arterial surface. In the second, secondary mechanical shaping of the injected scaffold casts is performed, with the aim of obtaining a regular large hollow structure.

The in situ channeling and shaping of the solidified material is performed using innovative technology (i.e., catheters) and mechanical atherectomy devices commonly used in clinical practice. In one method, an appropriately modified atherectomy device removes the diseased segment and is also be used to channel and shape the scaffold cast.

### EXAMPLE 3

### Approach A: A structured SIL or SIEL - Synthesis and delivery

A cylindrical or spiral synthetic layer made in a thin (<100 µm) sheet or a fiber mesh construct (e.g., multiple layers to form a 3D porous structure) of a biocompatible, resorbable (natural or synthetic) polymer (e.g., BRP). The structure may be made of multiple layers of the same or differently functionalized polymers or in combination with winding fibers made of a thicker deployment or a different BRP is produced. Fiber meshes are synthesized directly on the delivery catheter (balloon or expandable catheters) by electro spinning or other deployment technology and are delivered allowing a complete and full contact with the vessel inner wall (**Figure 11**). Fibrin can be added in the structure or deployed in solution as a biological glue to increase adhesion of the device and avoid collapse or inward folding. The cylinders are stabilized with special metallic nets temporarily left in the artery (**Figure 3**).

Nanoporosity and functionalization of the scaffold are differentiated among the inner surface and the outer surface. The inner surface is less porous than the outer surface. The inner surface comprises intracellular signaling molecules for circulating cell receptors (e.g., a biomolecule comprising an REDV (Arg-Glu-Asp-Val) based adhesion domains). The outer surface has a greater porosity than the inner surface to accommodate growth and attachment of smooth muscle cells to the scaffold trabecular structure. The outer surface comprises specific signaling molecules (e.g., a biomolecule comprising RGD (Arg-Gly-Asp)-based adhesion domains to stimulate growth and attachment of smooth muscle cells to the trabecular structure. The differential inner and outer structure is generated via synthesizing and unifying two cylinders with different scaffold conformations (Figure 3). Other synthesizing technology may be used. In preferred aspects, sheet cylinders will have a variable diameters ranging from 2.5 to 40 mm

The synthetic layer will be preserved in a dry status or wet solution, sterilized and able to be preserved on the shelf. It will be preserved as a cylinder to be mounted on a delivery system. Alternatively, the device will be pre-mounted in a delivery system. The synthetic layer will be elastic, moldable by a balloon delivery system and capable of maintaining an expanded status (e.g., avoiding collapsing) by adhering to the walls of the inner layer of the blood vessel. Adherence may be assisted through the combination of or fibrin or biological glue with the synthetic layer. This fibrin or biological glue may be added as part of a composite materials scaffold adhered to its outer surface, or may be added to the synthetic layer or blood vessel during delivery.

The cylindrical device layer will maintain its manufactured shape before implantation (e.g., during shipment and storage before use) and after implantation. The implanted cylindrical device will attach to the native arterial wall and avoid collapse, rupture and migration. The elastic property of the scaffold material will allow the cylindrical device to comply with systolic expansion of the artery, thus assuring a three phase shape of the distal Doppler wave, as assessed at fixed intervals in *in vivo* experimentation by Doppler ultrasound probing. This structural characteristic will be retained by the thinner thickness of the scaffold sheet, ideally around 20 to 40 µm.

After implantation, the scaffold will be populated by host cells, smooth muscle cells migrating from the outer surface and endothelial precursor cells (EPC) or endothelial cells (EC) on the inner surface. Chronic inflammatory reaction and levels of full integration through reconstituting an intimal layer will be measured. Successful implantation of the device will resemble a native intima.

The synthetic layer will be delivered in desired position by surgical exposition, as in surgical arterial endoarterectomy intervention. Alternatively, devices will be delivered in desired position by endovascular delivery systems, e.g. introducers and balloon catheters. These ensure efficient and precise delivery while maintaining the integrity of the scaffolds in the whole process of deployment. The synthetic layers will be available dry or as a wet solution. Synthetic layers will be sterilized during the synthetic process and maintain sterility up to implantation. The synthetic layer comprises single or multiple fiber mesh sheets.

The synthetic layer will be delivered via a balloon catheter on which the device will be pre-mounted. The synthetic layer will be delivered without contacting surfaces of the arterial system before the target area. The delivery system will assure a precise delivery, through markers (e.g., radiopaque markers) set on it and precisely indicating the edges of a non-radiopaque device. Full release will be done without any attachment of the device with the delivery catheter/dilator or delivery introducer sheet (**Figure 4A**).

A system for granting can stent the synthetic layer temporarily. The system will be removed after adhesion of the synthetic layer to the arterial wall. This system will be formed by an expandable metallic mesh which will be left in place and retrieved by advancing a sheet. This metallic mesh is treated so that it does not to attach, break or displace the synthetic layer in any of its maneuvers (**Figure 4B**).

The mesh layers of the scaffold or pre formed cylinders comprise single or multiple fiber mesh sheets. The scaffold can be synthesized directly on the delivery balloon (**Figure 4A**) by electro spinning or synthesized as cylinder meshes (**Figure 4B**) and mounted on an expanding delivery system as a metallic net, to be retracted and removed at completion of the delivery.

### EXAMPLE 4

### Approach B: An injectable scaffold - Synthesis and delivery

We disclose temperature sensitive, resorbable, injectable, polymeric scaffolds as hydrogels, which are delivered in liquid status by specially designed catheters. The liquid undergoes a phase shift to a polymeric scaffold hydrogel after injection at body temperature.

The structure of these scaffolds (e.g., synthetic injectable layers) are modeled subsequently by balloon or callipered mechanical atherectomy devices so to reach the desired thickness and a coherent vessel lumen. Alternatively, a "double phase" deployment, to resemble the pre-formed synthetic layer described above will be performed. Special spraying catheters and molding compliant balloons are generated (**Figure 5**).

The synthetic layer is a temperature sensitive hydrogel applied on a specially designed delivery balloon in its liquid phase. The hydrogel will solidify over the deflated balloon in a defined quantity and width, calculated on the base of the final desired quantity and width in the diameter of inflated balloon and target artery. The temperature sensitive gel can be placed on the balloon by spraying machines which can stratify the two different layers of temperature sensitive hydrogels, containing different signal molecules, one, internal, with specific endothelial progenitors cells receptors (REDV), the other, external, with the connective smooth muscle cells specific receptors (RGD). The balloon will be inflated, when used, with cold saline to revert the hydrogel to a liquid or semi-liquid phase. The rehydrated scaffold squeezes between the inflated balloon and the arterial inner wall, where it will solidify to its final conformation in the artery.

This technology will be directed to the treatment of isolated as well as diffuse lesions, and deployment will be influenced by the length of the delivery systems. The synthetic injectable layer will range from 2 to 30 cm depending on the size of the lesion.

The hydrogel will be stable and preserved in a sterile vial at room or mild refrigerated temperature. From this vial, in the endovascular applications, the liquid phase polymer will be transferred aseptically to the delivery system where it can be left dehydrating. In open surgery application the delivery system will be a manual spraying system, as for nasal/oral applications. In one embodiment, the spraying caps will be locked to the preservation vial.

Once the hydrogel is deployed and solidified, the scaffold will be resistant to early and late migration, rupture, chopping with distal embolization. After implantation, the scaffold will be populated by host cells, smooth muscle cells migrating from the outer surface and endothelial precursor cells (EPC) or endothelial cells (EC) on the inner surface. Chronic inflammatory reaction and levels of full integration through reconstituting an intimal layer will be measured. Successful implantation of the device will resemble a native internal intima **(****Fig 11****).**

The hydrogel will be delivered in desired position by deploying with a spray device directly onto the arterial surface. Alternatively, the hydrogel will be delivered by an specifically endovascular delivery systems (e.g., an introducers or balloon catheter). The delivery method will which assure efficient and precise delivery to the arterial wall. The sterile hydrogel, in liquid phase, will be stored and transferred to spray systems aseptically.

### Injectable Scaffold Delivery Device and Method 1

The delivery system is a balloon catheter. The catheters will be designed having two double compliant balloons which will assure flow blockage below and above the arterial segment to be treated (**Figure 6**). The catheter portion between those two balloons has micro-holes which will allow the liquid phase RBP hydrogel (e.g., Elastin Like Recombinant Polymers (ELRP)) to be sprayed onto the arterial inner surface. The spraying segment will be produced in different lengths in order to comply with different lesions length, e.g., from 2 to 30 cm. This procedure may be performed in combination with a "temporary stenting" safe technology.

The scaffold is delivered in liquid phase by a windowed catheter segment between the two double compliant balloons. This designed delivery system allows safe delivery of a temperature-sensitive (e.g., undergoes a phase change at body temperature) or self-setting RBP hydrogel formulation of a scaffold , while blocking the flow in the arterial segment to be treated, to grant time for a phase change, solidification or reaction of the scaffold hydrogel.

### Injectable Scaffold Delivery Device and Method 2

Alternatively, the use of currently available technology as a wire with compliant balloon at its top (for example Guardwire®, Medtronic Inc) and a 2 lumen balloon (for example The Dual lumen Fogarty balloon®) will used to obtain blood flow exclusion in the segment of arterial wall to be treated. A catheter with holes in the distal portion for a wide ranging extension (from 1.5 to 30 cm length) will be inserted over the guardwire for spraying the injectable polymer (**Figure 7**).

### Injectable Scaffold Delivery Device and Method 3

Another alternative deployment system for a temperature sensitive hydrogel is via a three balloon catheter, having two proximal and distal, compliant, hemostatic balloons and one central balloon, which will be semi compliant, with different compliance characteristics in its part in order to fill spaces horizontally without increasing its nominal diameter (**Figure 8A**). This balloon will be coated by the temperature sensitive RBP hydrogel scaffold in the liquid, cold phase, and let the system worm up so to bring the scaffold to a gel phase, to a desired, calculated quantity and width (**Figure 8B**). This balloon will be inflated with cold liquid in order to bring the hydrogel scaffold back to a semi liquid phase, for which it will be pushed and squeezed by the dilating balloon, between the balloon surface and the inner arterial wall (not touched by the balloon having a diameter slightly superior to the maximal nominal diameter of the fully expanded balloon (**Figure 8C**). In this maneuver, at body temperature, the whole system will warm up, having the hydrogel scaffold regaining its gel phase, over the arterial inner face (**Figure 8D**). **Figure 8E** shows the scaffold hydrogel successfully deposited into a cylinder. The hydrogel is stained with methylene blue.

### EXAMPLE 5

### Functionalization of scaffolds to obtain smart and efficient substrates for tissue regeneration.

Scaffolds proven to be biocompatible are modified in order to improve their ability to act as biological glue, promote endothelial cells' migration and confluence, and to anchor circulating endothelial progenitor cells.

The aim of functionalization is to increase the bio-glue (i.e., adherent) features of the materials maximizing adhesion to extracellular matrix and cellular (endothelial, endothelial precursors and smooth muscle cells) adhesion molecules. The adherence of selected subsets of cells results in engineered tissue growth. While endothelial and endothelial precursors are expected to be taken from the pool of circulating blood cells, smooth muscle cells are expected to migrate from the remaining healthy part of medial layer. One aspect of functionalization can comprise use of bioactive polymers to inhibit uncontrolled proliferation of Smooth Muscle Cells from the media. Selected adherence is achieved, for example, with the application of bioactive polymers engineered to express RGD (Arg-Gly-Asp) or REDV (Arg-Glu-Asp-Val) based adhesion domains and/or the application of appropriate nanometer-size porosity to bioresorbable substrates, to construct devices aimed at improving endothelial cell adhesion. Both the inner and the outer surfaces of the scaffolds are functionalized.

Another aspect of functionalization concerns the strategy for material/host tissue adhesion in order to maintain adherence of the preshaped SIL or SIEL from Approach A to the blood vessel. The preshaped SIL or SIEL is a thin walled cylinder, and thus will not rely on radial force to maintain its structure. To this aim the strategy involves the application of micro-nanometer-sized porosity to the bioresorbable substrates. This functionalization technology improves endothelial cell adhesion. The functionalized materials described above increase the overall adhesive properties of the SIL or SIEL of this example.

### EXAMPLE 6

### Validation of blood vessel tissue engineering compositions and methods in vitro and in vivo.

All proposed materials undergo full *in vitro* characterization and are tested both by conventional toxicological methods and more in-depth material biocompatibility assessment, through investigation of the interaction of mononuclear cells, macrophages and endothelial cells, both in mono and co-culture, measuring the release of inflammatory and chemoattractive molecules, cellular phenotype, proliferation, extracellular protein expression, gene expression and also using conventional histological methods. Suitability for in situ tissue engineering is determined. The kinetics of cellular re-population and the functional properties of the cells within the biopolymer(s) is measured and the expression of adhesion molecules in the cells embedded in the biopolymer is analyzed. Adhesion, proliferation and activation status of endothelial cells, endothelial progenitor cells, and smooth muscle cells is evaluated. Histo-chemical analyses are used to identify the viable and functionally active cells through LDL uptake, Hoechst/TUNEL and Annexin V staining. The thrombogenicity of both the EC-seeded and unseeded functionalized biomaterials to be used for the lumen is evaluated by measuring platelet activation and contact activation through flow cytometry, coagulometry and the use of acetylated prothrombin platelet-containing plasma.

Proposed materials from approaches A and B are tested *in vivo* in animal models using New Zealand white male rabbits and Landrace pigs. There are 3 phases of the *in vivo* testing: model preparation phase, main testing phase and GLP grade animal testing.

### Phase 1 - Model Preparation Phase

The model preparation phase comprises the set up of the control model and the scaffold implantation techniques. The control model consists of the adaptation of a well established restenosis model by percutaneous transluminal angioplasty balloon-induced intimal denudation model of the iliac arteries and aorta. This model comprises two interventions. The first intervention is aimed at producing chronic obstructive lesions. The second intervention is the surgical removal of the induced obstructive intimal hyperplasia. This is achieved using endoarterectomy in the earlier experimental phase and by endovascular technology in the pre clinical Good Laboratory Practice experiments using mechanical or laser atherectomy. These techniques exert photochemical and photomechanical actions, with energy dissipation depth ranges around 50µm and induction of less trauma on the arterial outer wall in comparison with mechanical endoarterectomy. The ablation is extended further to the "healthy" middle part of the medial layer as in the concept of "radical debulking of the artery". This phase of preparation demonstrates the experimental model's feasibility, reliability and reproducibility.

In this first stage, this control model is challenged by an as yet unexplored model and will focus on the induction of arterial intima denudation by immune xeno reaction according to a new model proposed by F. Serino and S. Miyagawa. This is compared to the consolidated model of balloon intimal injury. This set up enables us to perform the lesion phase and the scaffold implantation phase in the same setting, with significant reduction of animal utilization and suffering. This model is shown to be reliable, efficient and competitive with the classical one, and therefore is the base for the following animal experimental phases.

### Phase 2 - Main Testing Phase

The main testing phase is a feasibility study and an efficacy study in small and large animal models (i.e., a pig). First, a feasibility study of the scaffold implantation in rabbits is carried out to assess the inflammatory response and thrombogenicity of scaffold candidates. This feasibility study is carried out on naked and functionalized materials with short term and long-term efficacy studies, conducted first in rabbit models. From the data, a pair of scaffold candidates from each approach is selected and further refined and scaled into human vessel sizes for the long-term experiments in large animal models.

**Figure 9** shows the fiber mesh of a bioresorbable scaffold (in this case recombinant elastin like polymer) delivered and self retaining on the native rabbit aorta. In figure 9A, the polymer mesh was applied without pressure on the native aorta. Arginylglycylaspartic acid (RGD peptides) on the outer surface of the scaffold bind strongly to the inner layer of the aorta. In figure 9B, the polymer mesh was deployed with a balloon inflated at low presure (2 atm). The results show complete and total adhesion of the bioresorbable scaffold to the arterial wall.

A hydrogel of Elastin like Polymer with RGD peptides on the outer surface was deployed on native rabbit aorta, by a balloon catheter, at low inflation pressure in absence of stretching force applied on tissue. The resulting binding of the hydrogel to the aorta is shown in **Figure 10****.** The thin layer of hydrogel is intact adapted and firmly attached to the arterial wall, acting as a synthetic internal elastic lamina. In **Figure 11****,** it is shown that an SIL/ SIEL produced as described in this application is synthesized, deployed and retained in an animal normal artery and retains similar aspect, dimension and elasticity of the natural (underlying) one.

## Claims

1. A synthetic layer scaffold for rebuilding a diseased intima layer of an artery, comprising:
a biocompatible, bioresorbable synthetic elastic intima layer deployable on an inner surface of a diseased artery,
wherein the synthetic elastic intima layer is a polymer layer with a thickness less than 0.004 inches (100 µm);
wherein the synthetic elastic intima layer has pores to allow the outer surface to adhere to the inner surface wall of the diseased artery upon delivery in the artery and to allow cell ingrowth upon the delivery in the artery, wherein the diameter of the pores is between 1 to 10,000 microns;
wherein the synthetic elastic intima layer is bio-absorbable over time upon the delivery in the artery; and
wherein the bio-absorbable synthetic elastic intima layer is capable of being replaced by a newly rebuilt intima layer as a result of the cell ingrowth and the adherence of the outer surface of the bio-absorbable synthetic elastic intima layer to the inner surface wall of the artery.

2. A synthetic layer scaffold according to Claim 1, wherein at least some of the pores form at least a partial gradient from one region of the polymer layer to the next.

3. A synthetic layer scaffold according to Claim 1 further comprising an anti-adhesion filler filling at least some of the pores.

4. A synthetic layer scaffold according to Claim 1, wherein the synthetic elastic intima layer comprises an absorbable polymer or copolymer selected from Elastin like Polymers (ELP), polyglycolic acid (PGA), polylactic acid (PLA), polycaprolactone, polyhydroxyalkanoate, polyfumarate and derivatives each thereof.

5. A synthetic layer scaffold according to Claim 1 further comprising a biological material selected from collagen, fibrin, and elastin.

6. A synthetic layer scaffold according to Claim 1, wherein the polymer layer has a thickness less than 0.002 inches (50 µm).

7. A synthetic layer scaffold according to Claim 1, wherein the polymer layer has a thickness of 0.001 inch (25 µm).

## Patentansprüche

1. Synthetisches Schichtgerüst zum Wiederaufbauen einer erkrankten Intima-Schicht einer Arterie, umfassend:
eine biokompatible, bioresorbierbare, synthetische, elastische Intima-Schicht, die auf einer Innenfläche einer erkrankten Arterie entfaltet werden kann,
wobei die synthetische, elastische Intima-Schicht eine Polymerschicht mit einer Dicke von weniger als 0,004 Zoll (100 µm) ist;
wobei die synthetische, elastische Intima-Schicht Poren aufweist, um zu ermöglichen, dass die Außenfläche bei der Abgabe in die Arterie an der Innerflächenwand der erkrankten Arterie haftet, und um ein Einwachsen von Zellen bei der Abgabe in die Arterie zu ermöglichen, wobei der Durchmesser der Poren zwischen 1 bis 10.000 Mikron beträgt;
wobei die synthetische, elastische Intima-Schicht im Laufe der Zeit bei der Abgabe in die Arterie bioabsorbierbar ist; und
wobei die bioabsorbierbare, synthetische, elastische Intima-Schicht durch eine neu aufgebaute Intima-Schicht infolge des Einwachsens der Zellen und der Anhaftung der Außenfläche der bioabsorbierbaren, synthetischen, elastischen Intima-Schicht an der Innenflächenwand der Arterie ersetzt werden kann.

2. Synthetisches Schichtgerüst nach Anspruch 1, wobei mindestens einige der Poren mindestens einen Teilgradienten von einem Bereich der Polymerschicht zur nächsten bilden.

3. Synthetisches Schichtgerüst nach Anspruch 1, ferner umfassend einen Antihaftfüllstoff, der mindestens einige der Poren füllt.

4. Synthetisches Schichtgerüst nach Anspruch 1, wobei die synthetische, elastische Intima-Schicht ein absorbierbares Polymer oder Copolymer umfasst, das aus Elastin-ähnlichen Polymeren (ELP), Polyglykolsäure (PGA), Polymilchsäure (PLA), Polycaprolacton, Polyhydroxyalkanoat, Polyfumarat und Derivaten davon ausgewählt ist.

5. Synthetisches Schichtgerüst nach Anspruch 1, ferner umfassend ein biologisches Material, das aus Kollagen, Fibrin und Elastin ausgewählt ist.

6. Synthetisches Schichtgerüst nach Anspruch 1, wobei die Polymerschicht eine Dicke von weniger als 0,002 Zoll (50 µm) aufweist.

7. Synthetisches Schichtgerüst nach Anspruch 1, wobei die Polymerschicht eine Dicke von 0,001 Zoll (25 µm) aufweist.

## Revendications

1. Échafaudage à base de couches synthétiques pour reconstruire une couche d'intima affectée d'une artère, comprenant :
une couche d'intima élastique synthétique, biocompatible, biorésorbable déployable sur une surface interne d'une artère affectée,
dans lequel la couche d'intima élastique synthétique est une couche polymère ayant une épaisseur inférieure à 0,004 pouce (100 µm) ;
dans lequel la couche d'intima élastique synthétique possède des pores permettant à la surface externe d'adhérer sur la paroi de surface interne de l'artère affectée après la pose dans l'artère et permettant une interposition des cellules après la pose dans l'artère, dans lequel le diamètre des pores est compris entre 1 et 10 000 microns ;
dans lequel la couche d'intima élastique synthétique est bioabsorbable avec le temps après la pose dans l'artère ; et
dans lequel la couche d'intima élastique synthétique bioabsorbable peut être remplacée par une couche d'intima récemment reconstruite en conséquence de l'interposition des cellules et de l'adhérence de la surface externe de la couche d'intima élastique synthétique bioabsorbable sur la paroi de surface interne de l'artère.

2. Échafaudage à base de couches synthétiques selon la revendication 1, dans lequel au moins certains des pores forment au moins un gradient partiel, d'une région de la couche polymère à la suivante.

3. Échafaudage à base de couches synthétiques selon la revendication 1, comprenant en outre une charge anti-adhérence remplissant au moins certains des pores.

4. Échafaudage à base de couches synthétiques selon la revendication 1, dans lequel la couche d'intima élastique synthétique comprend un polymère ou un copolymère absorbable choisi parmi des polymères de type élastine (ELP), l'acide polyglycolique (PGA), l'acide polylactique (PLA), la polycaprolactone, un polyhydroxyalcanoate, un polyfumarate et des dérivés de chacun de ceux-ci.

5. Échafaudage à base de couches synthétiques selon la revendication 1, comprenant en outre un matériau biologique choisi parmi du collagène, de la fibrine, et de l'élastine.

6. Échafaudage à base de couches synthétiques selon la revendication 1, dans lequel la couche polymère a une épaisseur inférieure à 0,002 pouce (50 µm).

7. Échafaudage à base de couches synthétiques selon la revendication 1, dans lequel la couche polymère a une épaisseur de 0,001 pouce (25 µm).
